# EUROPÄISCHE PATENTANMELDUNG

(11) **EP 0 628 535 A1**
(43) Veröffentlichungstag der Anmeldung: **14.12.1994**
(21) Anmeldenummer: 94107822.2
(22) Anmeldetag: 20.05.1994
(51) Int. Cl.: C07C 209/26, C07D 213/38, C07C 227/08

(54) **Verfahren zur Herstellung primärer Amine aus Aldehyden**

(30) Priorität: 07.06.1993 AT 1100/93
(71) Anmelder: DSM Chemie Linz GmbH, 4021 Linz (AT)
(72) Erfinder: Kos, Carlo, Dr., A-4060 Leonding (AT); Hebesberger, Friedrich, A-4073 Wilhering (AT); Artner, Eduard, Dipl.-Ing., A-4040 Linz (AT); Kloimstein, Engelbert, Ing., A-4070 Eferding (AT); Haar, Robert, A-4210 Engerwitzdorf (AT); Lust, Ernst, A-4020 Linz (AT)
(74) Vertreter: Kunz, Ekkehard, Dr.

(57) **Zusammenfassung**

Verfahren zur Herstellung primärer Amine aus Aldehyden durch Vermischen eines Aldehyds mit einem Verdünnungsmittel, wobei im Falle eines Alkohols oder von Wasser als Verdünnungsmittel die Mischungstemperatur höchstens 5 °C beträgt, um die Bildung des Hemiacetals oder des Aldehydhydrats zu verhindern und Inkontaktbringen der Mischung mit einer Mischung aus Ammoniak, Wasserstoff und Hydrierkatalysator, wodurch die Bildung eines Imins verhindert wird, bei Temperaturen von 60 bis 180 °C und Drücken von 20 bis 60 bar, wobei pro Mol Aldehydgruppe mindestens 15 Mol Ammoniak verwendet werden, sowie Vorrichtung zur Durchführung des Verfahrens.

## Beschreibung

Primäre Amine und Diamine, wie beispielsweise Hexamethylendiamin stellen wichtige Bausteine bei der Synthese organischer Verbindungen dar. Ihre Herstellung kann beispielsweise durch reduktive Aminierung von Aldehyden oder von anderen Produkten, die aus der Ozonolyse olefinischer Doppelbindungen stammen, erfolgen. Obwohl die reduktive Aminierung von Carbonylverbindungen seit langem bekannt ist, konnte bisher kein Verfahren gefunden werden, bei dem die Bildung sekundärer und tertiärer Amine verhindert werden konnte und bei dem die primären Amine nach kurzer Reaktionszeit rein und in hohen Ausbeuten anfallen.

So ist in J.Am. Chem. Soc. 70 (1948), pp. 1315 - 1316 die reduktive Alkylierung von Ammoniak mit Ketonen und Aldehyden in Gegenwart von Ammoniumchlorid, absolutem, ammoniakgesättigtem Methanol, Wasserstoff und Platinoxid als Katalysator beschrieben. Die Ausbeuten betragen dabei 10 bis höchstens 69 %.

Aus US 2,657,240 ist bekannt, Cyclohexen in einem gesättigten, aliphatischen Alkohol zu ozonisieren und das Ozonolyseprodukt mit einem Überschuß von Ammoniak in Gegenwart eines Hydrierkatalysators und anschließend oder gleichzeitig mit Wasserstoff zu behandeln, wobei, allerdings nur in geringen Ausbeuten und nach langer Reaktionszeit, Hexamethylendiamin entsteht.

Die Reaktion olefinischer Doppelbindungen mit Ozon und Umsetzung des Ozonolyseproduktes mit Ammoniak und Wasserstoff in Gegenwart eines Hydrierkatalysators zur Herstellung von Aminen ist auch in Pollart und Miller, J. Org. Chem. 27, (1962), pp. 2392-94 beschrieben. Der Umsetzungsgrad der verschiedenen Olefine beträgt aber nur 12,5 bis höchstens 71 %. Eine Verbesserung dieser Methode soll gemäß White et al, Tetrahedron Letters No. 39, (1971), pp. 3591-3593 mit Hilfe einer Dreistufenmethode, nämlich a) Ozonolyse eines Olefins in einem Alkohol b) katalytische Hydrierung der Ozonolyseprodukte c) reduktive Aminierung unter Verwendung eines Rhodiumkatalysators und Temperaturen von 50 bis 60 °C oder eines Raney Nickel Katalysators bei 80 bis 100 °C erreicht werden. Aber auch in diesem Fall betragen die Ausbeuten höchstens 80 %.

In Diaper and Mitchell, Canadian Journal of Chemistry, Vol. 40 (1962), pp. 1189-1195 ist die reduktive Aminierung der Ozonolyseprodukte von Alkenolen, Alkencarbonsäureestern, Alkencarbonsäure und Alkenamiden, wobei Aminoalkanole, Aminoalkancarbonsäureester, Aminoalkancarbonsäuren und Aminoalkanamide entstehen, beschrieben. Die Ausbeuten lassen aber auch hier zu wünschen übrig und die Reaktionszeiten sind lang.

In DE-PS 824 492 ist ein Verfahren zur Herstellung aliphatischer Diamine mit langer Kette durch Einbringen der entsprechenden Dialdehyde in eine Reaktionsmischung bestehend aus Ammoniak, einem Hydrierkatalysator und Wasserstoff bei hohen Drucken angegeben. Gemäß der Beschreibung soll möglichst kein Verdünnungsmittel verwendet werden. Gemäß der Beispiele wird allerdings Wasser als Verdünnungsmittel für den verwendeten Dialdehyd eingesetzt. Bei dieser Art der Reaktionsführung verläuft aber die Reaktion nur langsam und unter Bildung von Nebenprodukten.

In Houben Weyl, Methoden der organischen Chemie, Band XI, 1, 1957, Seite 604 ist die Herstellung von Propylamin durch Einbringen von Propionaldehyd in eine Mischung von Methanol, Raney-Nickel, flüssiges Ammoniak und Wasserstoff mit einer Temperatur von 138°C und Erhöhen der Reaktionstemperatur unter Aufrechterhaltung eines Druckes von 40 bis 140 at durch Ergänzung des verbrauchten Wasserstoffes beschrieben. Dabei entstehen aber relativ hohe Anteile an Nebenprodukten.

In DE 26 47 317 ist ein zweistufiges Verfahren zur Herstellung von gesättigten oder ungesättigten, aliphatischen, linearen alpha,omega-Diaminen durch redukte Aminierung der entsprechenden alpha,omega-Dialdehyde geoffenbart, wobei die Aldehyde durch Zugabe von Ammoniak in einer ersten Stufe zu den Diiminen und diese daraufhin mit Hilfe von Wasserstoff und einem Katalysator zu den Diaminen reduziert werden. Die Ausbeuten betragen nach diesem Verfahren zwar bis zu 90 %, eine zweistufige Arbeitsweise bedeutet aber bei einem industriell durchgeführten Verfahren einen enormen Mehraufwand an Apparaturen. In EP-A-0 400 426 wird ebenfalls ein zweistufiges Verfahren zur Umsetzung eines alpha,omega-Dialdehyds zu den entsprechenden primären alpha,omega-Diaminen vorgestellt, bei dem der Dialdehyd in einer ersten Stufe mit einem primären Amin, z. B. Butylamin, und das Reaktionsprodukt in einer zweiten Stufe in Gegenwart von Ammoniak, Wasserstoff und einem Hydrierkatalysator umgesetzt wird. Abgesehen von der zweistufigen Verfahrensweise besitzt dieses Verfahren auch den Nachteil, daß das primäres Amin zuerst bereitgestellt und später wieder aus dem Reaktionsgemisch entfernt werden muß.

In FR 2,656,864 ist ein zweistufiges Verfahren zur Herstellung aliphatischer Diamine aus den entsprechenden Dialdehyden beschrieben, bei dem in der ersten Stufe der Dialdehyd mit einem Alkohol unter solchen Bedingungen umgesetzt wird, daß das Di-Halbacetal gebildet wird, das in einer zweiten Stufe einer Aminier- und Hydrierreaktion mittels Versetzen mit Ammoniak, Wasserstoff und einem Hydrierkatalysator unterworfen wird. Die Selektivität der Reaktion soll 89 % betragen, die beschriebene Ausbeute an Diaminooctan ausgehend von Octandial beträgt tatsächlich aber nur 52 %. Überdies beschränken sich die beschriebenen Verfahren, die eine etwas bessere Ausbeute aufweisen, auf die Herstellung von Diaminen.

Es wurde nun unerwarteterweise gefunden, daß sowohl Monoaldehyde, als auch organische Verbindungen mit mehr als einer Aldehydgruppe einstufig und in sehr hohen Ausbeuten an sehr reinem Produkt, wobei die Reaktionszeiten verblüffend kurz sind, reduktiv aminiert werden können, wenn man einen Aldehyd und ein Verdünnungsmittel zusammenführt, wobei im Falle eines Alkohols oder von Wasser als Verdünnungsmittel die Mischung bei so tiefen Temperaturen zusammengeführt wird, daß in der Mischung kein Hemi- oder Halbacetal oder kein Aldehydhydrat gebildet wird und wenn man die Mischung unmittelbar nach dem Mischen praktisch gleichzeitig mit Ammoniak, einem Hydrierkatalysator und Wasserstoff in Kontakt bringt, wodurch die Bildung eines Imins verhindert wird, sodaß der Ammoniak und der Wasserstoff direkt mit der Aldehydgruppe und nicht mit einem Derivat davon in Kontakt kommen.

Gegenstand der Erfindung ist daher ein Verfahren zur Herstellung primärer Amine aus Aldehyden in Gegenwart eines Verdünnungsmittels, Ammoniaks, wobei pro Mol Aldehydgruppe mindestens 15 Mol Ammoniak verwendet werden, eines Hydrierkatalysators, Wasserstoffs und Isolierung des gebildeten Amins aus dem Reaktionsgemisch, das dadurch gekennzeichnet ist, daß, ein Aldehyd mit einem Verdünnungsmittel, wobei im Falle eines Alkohols oder von Wasser als Verdünnungsmittel die Mischungstemperatur höchstens 5°C beträgt, gemischt wird, sodaß sich kein Hemiacetal oder Aldehydhydrat bildet und die Mischung unmittelbar danach, praktisch gleichzeitig mit Ammoniak, Wasserstoff und dem Hydrierkatalysator bei Temperaturen von 60 bis 180°C und Drücken von 20 bis 60 bar in Kontakt gebracht wird.

Zur Durchführung des erfindungsgemäßen Verfahrens wird ein Aldehyd in einem Verdünnungsmittel gelöst.

Unter Aldehyd sind aliphatische oder aromatische Monoaldehyde und aliphatische Verbindungen mit mehr als einer Aldehydgruppe, insbesondere Dialdehyde zu verstehen. Aliphatische Aldehyde bedeutet gesättigte oder ungesättigte, geradkettige, verzweigte oder cyclische Alkane, Alkene oder Alkine, die entweder nur durch eine oder durch mehrere Aldehydgruppen und zusätzlich durch unter den Reaktionsbedingungen inerte Gruppen wie z. B. Phenyl-, Alkohol-, Alkoxy-, Amino-, Carbonsäure-, Carbonsäureester-, Carbonsäureamidgruppen substituiert sein können. Die Phenylgruppe kann ihrerseits durch Alkylgruppen oder durch oben angegebene funktionelle Gruppen substituiert sein. Aromatische Monoaldehyde sind unsubstituierte oder durch unter den Reaktionsbedingungen inerte Gruppen, wie beispielsweise durch Alkylgruppen und/oder oben angegebene Gruppen, substituierte Phenyl-, Naphthylgruppen oder heteroaromatische Ringe wie Pyrrol-, Furan-, Thiophen- oder Pyridinringe, bei denen eines der Wasserstoffatome an einem der Kohlenstoffatome durch eine Aldehydgruppe ersetzt ist.
Bevorzugt bedeuten aliphatische Aldehyde Alkanale, Alkenale, Alkandiale oder Alkendiale mit 4 bis 22 C-Atomen, die unsubstituiert oder durch Alkohol-, Alkoxy-, Amino-, Carbonsäure-, Carbonsäureestergruppen substituiert und die bevorzugt geradkettig sind. Als aromatische Aldehyde sind Benzaldehyde, die unsubstituiert, oder durch Alkyl-, Alkoxy-, Alkohol-, Amino-, Carbonsäure-, Carbonsäureestergruppen substituiert sind, bevorzugt. Bevorzugte heteroaromatische Aldehyde sind Pyrrol- oder Pyridinaldehyde.
Bevorzugte Alkyl- und Alkoxygruppen besitzen 1 bis 6 , besonders bevorzugt 1 - 3 C-Atome. Aminogruppen sind unsubstituiert oder durch Alkyl- oder Phenylgruppen substituiert.

Als Verdünnungsmittel können unter den Reaktionsbedingungen inerte Verdünnungsmittel, beispielweise aliphatische Kohlenwasserstoffe, wie Hexan, Pentan, aromatische Kohlenwasserstoffe, wie Toluol, Xylole, Ether, wie iso-Propylether, Methyl-tert.Butylether, Pyridin, Wasser und Alkohole oder Mischungen solcher Verdünnungsmittel eingesetzt werden. Bevorzugt wird ein Alkohol, Toluol, Methyl-tert.Butylether, Tetrahydrofuran, Dioxan, Pyridin, Wasser, besonders bevorzugt ein aliphatischer Alkohol mit 1 bis 8 C-Atomen, beispielsweise Methanol, Ethanol, iso-Propanol, Hexanol, Octanol eingesetzt, wobei Alkohole mit 1 bis 3 C-Atomen ganz besonders bevorzugt sind. Das Verdünnungsmittel wird im Überschuß zum Aldehyd eingesetzt, bevorzugt im 5 bis 30 fachen Überschuß bezogen auf das Gewicht des Aldehyds. Der verwendete Aldehyd muß im Verdünnungsmittel löslich sein.

Unerwarteterweise wurde gefunden, daß es von großer Bedeutung für Ausbeuten, Reinheit und Reaktionszeit bei der reduktiven Aminierung von Aldehyden ist, daß ein Verdünnungsmittel verwendet wird und daß dabei das Ammoniak und der Wasserstoff mit der Aldehydgruppe selbst und nicht mit dem Hemiacetal, das sich normalerweise beim Kontakt eines Aldehyds mit einem Alkohol bildet, oder mit dem Aldehydhydrat, das sich normalerweise beim Kontakt eines Aldehyds mit Wasser bildet, oder mit dem Imin, das sich normalerweise beim Kontakt eines Aldehyds mit Ammoniak ohne Wasserstoff und Hydrierkatalysator bildet, reagieren.
Es hat sich gezeigt, daß die Bildung des Hemiacetals oder des Aldehydhydrats bei Mischungstemperaturen von Aldehyd und Alkohol und/oder Wasser von unter 5°C verhindert wird, wobei im Falle von Wasser der Gefrierpunkt durch das Vermischen mit dem Aldehyd im allgemeinen hinaufgesetzt wird. Der Gefrierpunkt des Wassers kann auch durch Zugabe eines zweiten, organischen, mit Wasser mischbaren Verdünnungsmittels hinaufgesetzt werden. Nach dem Vermischen wird die Mischung möglichst schnell und praktisch gleichzeitig mit dem Ammoniak, dem Hydrierkatalysator und dem Wasserstoff in Kontakt gebracht, wodurch die Bildung des Imins verhindert wird.

Ammoniak, Wasserstoff und Hydrierkatalystor werden in die Mischung aus Aldehyd und Verdünnungsmittel wie üblich entweder zugegeben oder die Mischung aus Aldehyd und Verdünnungsmittel wird in eine Mischung des Ammoniaks mit dem Wasserstoff, gegebenenfalls dem Verdünnungsmittel und dem Hydrierkatalysator eingebracht.

Vorzugsweise wird Ammoniak mit dem Hydrierkatalysator und Wasserstoff und gegebenenfalls mit dem Verdünnungsmittel vermischt. In diese Mischung, die auf Temperaturen von 60 bis 200 °C, bevorzugt auf 80 bis 130 °C erhitzt wird, wird die Mischung aus Aldehyd und Verdünnungsmittel eingebracht. Die Reaktion erfolgt dabei unter Druck, wobei Drücke von 20 bis 60 bar, bevorzugt von 40 bis 60 bar verwendet werden. Der Druck setzt sich im wesentlichen aus den Partialdrücken des Ammoniaks, des verwendeten Verdünnungsmittels und des Wasserstoffs zusammen. Da der Partialdruck des Ammoniaks unter den Reaktionstemperaturen sehr hoch ist, wird das Ammoniak bevorzugt mit dem Verdünnungsmittel vorgelegt, wodurch eine Senkung des Gesamtdruckes erreicht wird.
Unter diesen Reaktionsbedingungen entsteht im Reaktionsgefäß eine flüssige und eine gasförmige Phase.

Das Ammoniak wird in hohem, molaren Überschuß, bezogen auf den Aldehyd, eingesetzt. Pro Mol Aldehyd werden mindestens 15, bevorzugt 20 bis 50, ganz bevorzugt 20 bis 35 Mol Ammoniak eingesetzt. Als Hydrierkatalysator dienen übliche, bei der reduktiven Aminierung von Carbonylverbindungen verwendete Katalysatoren, wie Nickel-, Kobalt-, Platin-, Palladiumkatalysatoren oder Verbindungen solcher Metalle, beispielsweise Oxide, die miteinander und/oder mit anderen Metallen oder Metallverbindungen, beispielsweise Eisen, Rhodium, Kupfer legiert, durchsetzt, oder beschichtet sein können. Der Katalysator kann dabei als solcher, aufgebracht auf einen üblichen Träger, oder als Festbettkatalysator oder Monolithkatalysator eingesetzt werden.
Im allgemeinen werden pro Mol Aldehyd mindestens 5,0 g Katalysator verwendet. Da die optimale Menge des Katalysators von seiner Effektivität abhängt, kann es aber von Vorteil sein, größere oder kleinere Katalysatormengen einzusetzen. Der optimale Katalysator und die optimale Katalysatormenge ist leicht durch Vorversuche für jeden Aldehyd zu ermitteln.
Beim Einsatz ungesättigter Aldehyde wird ein Katalysator verwendet, der eine C-C-Mehrfachbindung unter den Reaktionsbedingungen nicht angreift. Solche Katalysatoren, wie z. B. Nickelkatalystoren, sind bekannt.
Der Wasserstoff wird wie üblich in die Reaktionsmischung eingebracht, vorteilhafterweise wird der Wasserstoff auf die flüssige Phase aufgedrückt. Dabei wird ein Wasserstoffpartialdruck von mindestens 3 bar, bevorzugt von mindestens 5 bar angewendet und aufrechterhalten. Höhere Wasserstoffdrücke können dabei von Vorteil sein.

Bei Durchführung der erfindungsgemäßen Reduktion wird jede Aldehydgruppe, die in der Reaktionsmischung vorhanden ist, in eine Aminogruppe übergeführt. Aufgrund der Erfahrung mit herkömmlichen Verfahren, ist die nötige Reaktionszeit verblüffend kurz. Die Reaktion ist völlig unerwartet im allgemeinen innerhalb einer halben Stunde und in vielen Fällen schon nach einigen Minuten abgeschlossen.

Die Reaktion kann kontiuierlich oder diskontinuierlich durchgeführt werden und wird bevorzugt kontinuierlich durchgeführt.

Das Ende der Reaktion, bzw. die Verweilzeit bei kontinuierlicher Reaktionsführung, wird, wie üblich, bevorzugt chromatographisch, festgestellt. Nach beendeter Reaktion wird das Ammoniak und das Verdünnungsmittel nach Abtrennung vom Katalysator aus dem Reaktionsgemisch entfernt. Im Rückstand befindet sich das aus dem Aldehyd gebildete Amin in großer Reinheit und in Ausbeuten, die üblicherweise weit über 90 % betragen. Gegebenenfalls kann noch ein Reinigungsschritt z. B. durch Chromatographie, Destillation angeschlossen werden. Nicht destillierbare Amine können auch durch Überführung in eines ihrer Salze, beispielsweise in das Hydrochlorid, Hydrosulfat, Acetat weiter gereinigt werden.

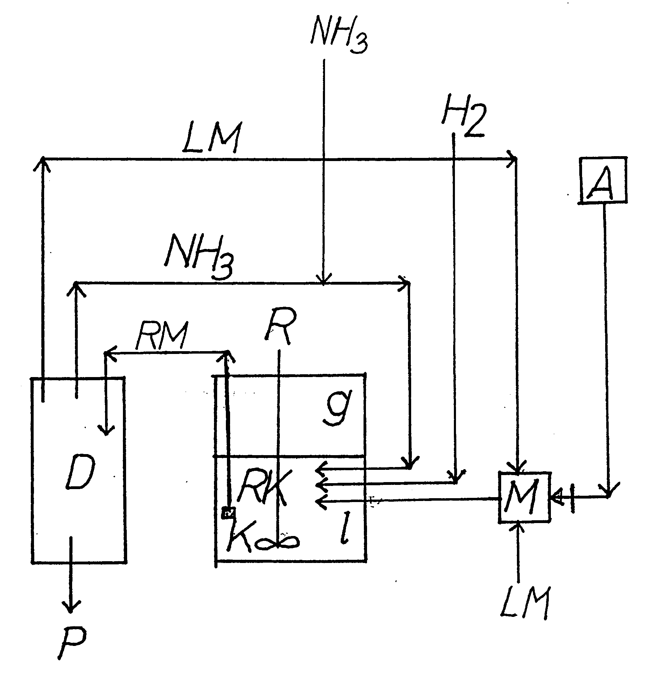

Ein weiterer Gegenstand der Erfindung ist eine Vorrichtung zur Durchführung des Verfahrens gemäß der Zeichnung.
In der Zeichnung bedeuten A eine kühlbare Vorrichtung zur Zuführung des Aldehyds, der umgesetzt werden soll, LM eine kühlbare Vorrichtung zur Beförderung des Verdünnungsmittels, M ein Mischgefäß, das gekühlt werden kann, RK das Reaktionsgefäß, das drucksicher ist, beheizt werden kann, eine Durchmischungsvorrichtung R und den Katalysator K enthält, sowie Zuleitungen für Wasserstoff H₂, Ammoniak NH₃, das Verdünnungsmittel LM und den Aldehyd A und eine Austragsvorrichtung für das ausreagierte Gemisch RM, das sich aus dem Produkt, Ammoniak, gegebenenfalls Wasserstoff und Verdünnungsmittel zusammensetzt. D bedeutet eine Aufarbeitungsanlage, vorzugsweise eine Destillationsanlage, in der der flüchtige Ammoniak und gegebenenfalls Wasserstoff aus dem ausreagierten Gemisch ausgetragen, das Verdünnungsmittel abgedampft und das Produkt isoliert wird.

Zur Durchführung der Reaktion wird der Aldehyd A im Mischgefäß M in dem Verdünnungsmittel LM gelöst, sodaß die Temperatur der Lösung 5°C nicht übersteigt. Im Reaktionsgefäß RK wird das Ammoniak NH₃, Wasserstoff H₂ und ein Nickelkatalysator bei Temperaturen von 80 bis 150°C und einen Druck von 20 bis 60 bar vorgelegt. Unter diesen Bedingungen bildet sich eine gasförmige Phase g und eine flüssige Phase I. Wird der Ammoniakdruck zu hoch, kann Verdünnungsmittel zugegeben werden. Da sich das Ammoniak im Verdünnungsmittel zumindest teilweise löst oder sich damit mischt, wird der Gesamtdruck auf diese Weise gesenkt. Die Lösung des Aldehyds im verwendeten Verdünnungsmittel wird in diese flüssige Phase I eingetragen. Die Durchmischung der flüssigen und gasförmigen Phase erfolgt durch eine Durchmischungsvorrichtung, etwa durch ein Rührwerk. Dabei erfolgt die Reaktion im allgemeinen praktisch augenblicklich und es sind im allgemeinen nur sehr kurze Verweilzeiten der Reaktanten im Reaktionsgefäß nötig. Weniger reaktive Aldehyde benötigen eine längere Verweilzeit. Die Reaktionszeit, d.h. die optimale Verweilzeit, nämlich die Geschwindigkeit der Zulieferung der Reaktanten und der Austrag des ausreagierten Gemisches kann für jeden Aldehyd durch Vorversuche problemlos ermittelt werden. Das ausreagierte Reaktionsgemisch RM wird ausgetragen, wobei ein gleichzeitiger, unerwünschter Austrag des Katalysators K, beispielsweise durch Verwendung einer Fritte, verhindert wird. Das Reaktionsgemisch RM kann sodann einer Destillationsanlage D zugeführt werden, in der das Ammoniak, gegebenenfalls der Wasserstoff und das Verdünnungsmittel vom Produkt abgetrennt und dem Reaktionsgefäß wieder zugeführt werden, wobei das Verdünnungsmittel gegebenenfalls vorher noch einer Reinigung, etwa zur Entfernung des gebildeten Reaktionswassers, unterzogen wird, oder das ausreagierte Reaktionsgemisch RM wird nach dem Austragen unabhängig von der Vorrichtung wie üblich aufgearbeitet. Es hat sich herausgestellt, daß für eine optimale Umsetzung eine pulsierende, kontinuierliche Änderung der Aldehydkonzentration in der Reaktionsmischung vorteilhaft sein kann. Es wird daher bevorzugt in bestimmten Zeitabständen, die von Fall zu Fall leicht zu ermitteln sind, anstatt der Mischung aus Aldehyd/Verdünnungsmittel nur Verdünnungsmittel in das Reaktionsgefäß eingetragen und die Zugabe von Aldehyd kurzzeitig unterbrochen.

Auf die beschriebene Art und Weise werden sehr reine primäre Amine in hohen Ausbeuten und in extrem kurzer Reaktionszeit hergestellt. Die Erfindung stellt daher eine Bereicherung der Technik dar.

### Beispiel 1

In einen 21 Druckreaktor wurden 215,6 g Ammoniak (12,65 Mol), 150 ml Methanol und 5 g Nickel Katalysator Ni 5256 der Firma Engelhardt mit ca. 55 - 60% Nickel auf SiO₂/Al₂O₃ eingetragen und auf eine Temperatur von 11 0°C erhitzt. Durch Aufdrücken von Wasserstoff wurde ein Gesamtdruck von 58 bar eingestellt. Dabei bildete sich eine flüssige und eine gasförmige Phase.
6,77 g 1,8-Octandialdehyd mit einer Reinheit von 97,3% wurden in 50 ml Methanol bei Temperaturen von -5 bis 0°C gelöst. Diese Lösung, die auf einer Temperatur von etwa 0°C gehalten wurde, wurde innerhalb von 25 Minuten in die gerührte, flüssige Phase des Druckreaktors eingetragen. Der Wasserstoffverbrauch, der gemessen wurde, war etwa 5 Minuten nach Ende des Eintragens der Lösung beendet, und das Reaktionsgemisch wurde über eine Fritte, die den Katalysator zurückhielt, aus dem Reaktor entnommen. Aus dem Reaktionsgemisch wurde das Ammoniak abgedampft und das Methanol und das gebildete Reaktionswasser abdestilliert. Der Rückstand von 7,4 g wurde im Kugelrohr destilliert.
Dabei wurden 6,42 g 1,8 Octandiamin, das sind 95% der Theorie bezogen auf reinen Aldehyd, mit einer Reinheit von 99% erhalten.

### Beispiel 2

wurde wie im Beispiel 1 beschrieben, aber kontinuierlich durchgeführt. Nach Vorlage von Ammoniak, Katalysatorsuspension und Wasserstoff wurden dazu 1440 ml/Stunde Ammoniak, 800 ml/Stunde Methanol und 28,4 ml/Stunde 1,8-Octandialdehyd in die flüssige Phase des Druckreaktors eingetragen wobei ein Gesamtdruck von 58 bar durch Wasserstoffzufuhr aufrechterhalten wurde. Gleichzeitig wurden 2228,4 ml/h Reaktionslösung aus dem Reaktor ausgetragen. Der Austrag erfolgte durch Entnahme von jeweils etwa 557 ml Reaktionslösung nach jeweils 25 Minuten. Jede Fraktion wurde gesondert aufgearbeitet. Dabei wurden die in Tabelle 1 zusammengestellten Ergebnisse erhalten:

**Tabelle 1**

| **Cyclus** | **Abdampfrückstand in Gramm** | **Diamin in Gramm** | **Ausbeute % der Theorie** |
|---|---|---|---|
| 1 | 5,3 | 4,9 | 71,4 |
| 2 | 7,4 | 6,2 | 90,8 |
| 3 | 8,9 | 7,3 | 106,4 |
| 4 | 3,2 | 1,4 | 20,4 |
| 5 | 3,4 | 0,8 | 11,7 |
| Summe | 28,2 | 20,6 | 60,1 |

### Beispiel 3

wurde wie Beispiel 2 durchgeführt, wobei aber nach Vorlage von Ammoniak, Katalysator und Wasserstoff in den Druckreaktor kontinuierlich 2500 ml Ammoniak/h, 1500 ml/h Methanol und 83,8 ml/h 1,8-Octandialdehyd in die flüssige Phase des Druckreaktors eingetragen wurden. Nach 24 Minuten wurde die Zufuhr des 1,8-Octandialdehyds jeweils gestoppt und jeweils 12 Minuten lang je 500 ml Ammoniak und 150 ml Methanol eingetragen. Gleichzeitig wurden kontinuierlich alle 36 Minuten 2283,5 ml der Reaktionsmischung aus dem Reaktor entnommen, die jeweils gesondert aufgearbeitet wurden.
Dabei wurden die in Tabelle 2 zusammengestellten Ergebnisse erhalten:

**Tabelle 2**

| **Cyclus** | **Abdampfrückstand in Gramm** | **Diamin in Gramm** | **Ausbeute % der Theorie** |
|---|---|---|---|
| 1 | 34,3 | 30,3 | 93,5 |
| 2 | 35,7 | 29,4 | 90,8 |
| 3 | 33,2 | 30,4 | 93,8 |
| 4 | 35,1 | 29,7 | 91,7 |
| 5 | 32,5 | 30,0 | 92,6 |
| Summe | 170,8 | 149,8 | 92,4 |

### Beispiele 4 und 5

wurden auf die im Beispiel 1 beschriebene Art und Weise, aber unter Verwendung von 150 ml Toluol bzw. von 150 ml Methyl.-tert.Butylether anstatt 150 ml Methanol im Druckreaktor und von 50 ml Toluol bzw. 50 ml Methyl.-tert.Butylether anstatt 50 ml Methanol als Lösungsmittel für 1,8-Octandialdehyd ausgeführt, wobei eine Ausbeute an 1,8-Octandiamin von 90,1 bzw. 87,8% der Theorie erhalten wurde

### Beispiel 6

wurde auf die im Beispiel 1 beschriebene Art und Weise, aber unter Verwendung von 1,12-Dodecandialdehyd einer Reinheit von 98,3% anstatt 1,8-Octandialdehyd ausgeführt. Dabei wurden 9,75 g 1,12-Dodecandiamin, das ist eine Ausbeute von 97% der Theorie bezogen auf den Reinaldehyd in einer Reinheit von 98% erhalten.

### Beispiel 7

wurde wie im Beispiel 3 beschrieben durchgeführt, wobei jedoch 5000 ml/h Ammoniak, 250 g/h 1,12-Dodecandialdehyd und 5000 ml/h Methanol eingetragen wurden, wobei gleichzeitig kontinuierlich alle 12 Minuten 2050 ml der Reaktionsmischung ausgetragen wurden. Die durchschnittliche mittlere Verweilzeit der Reaktanten, das ist jene Zeit, in der das Reaktorvolumen praktisch erneuert ist, betrug dabei 3.98 Minuten. Die Ausbeute des erhaltenen 1,12-Dodecandiamins betrug über 97%, die Reinheit etwa 98%.

### Beispiele 8 bis 22

Die Beispiele 8 bis 22 wurden nach der im Beispiel 3 beschriebenen Art und Weise, aber unter Verwendung verschiedener Aldehyde A als Ausgangsmaterial, verschiedener Aldehydkonzentrationen c (Mol/l) und verschiedener Verweilzeiten h (in Minuten) der Reaktanten durchgeführt. Dabei wurden die in Tabelle 3 zusammengestellten Ergebnisse erhalten:

**Tabelle 3**

| **Beispiel** | **A** | **c** | **V** | **h** |
|---|---|---|---|---|
| 8 | CH₃(CH₂)₅CHO | 0,35 | MeOH | 25 |
| 9 | CH₃(CH₂)₆CHO | 0,40 | MeOH | 25 |
| 10 | H₃COOC-(CH₂)₆-CHO | 0,80 | MeOH/Toluol | 25 |
| 11 | H₃COOC-(CH₂)₆-CHO | 0,80 | Pyridin | 25 |
| 12 | OH-CH₂(CH₂)₆-CHO | 0,60 | MeOH | 25 |
| 13 | (H₃CO)₂-CH-(CH₂)₆-CHO | 0,50 | MeOH | 25 |
| 14 | CH₃(CH₂)₇-CHO | 0,60 | MeOH | 11 |
| 15 | NaOOC-(CH₂)₇-CHO | 0,80 | MeOH | 25 |
| 16 | NaOOC-(CH₂)₁₀-CHO | 0,10 | H₂O/Pyridin | 4 |
| 17 | Benzaldehyd | 0,35 | MeOH | 40 |
| 18 | Vanillin | 0,15 | MeOH | 60 |
| 19 | Piperonal | 0,30 | MeOH | 60 |
| 20 | Veratrumaldehyd | 0,15 | MeOH | 40 |
| 21 | Pyridin-2-al | 0,50 | MeOH | 11 |
| 22 | Pyridin-4-al | 0,25 | MeOH | 60 |

Nach Aufarbeitung wurden die den Aldehyden und den Dialdehyden entsprechenden Amine und Diamine in einer Reinheit, die der Reinheit des als Ausgangsmaterial verwendeten Aldehyds praktisch äquivalent war (aus 100 % reinem Aldehyd entstanden praktisch 100 % reines Amin) und in Ausbeuten von jeweils über 90 % erhalten.

Die Reinheit der Amine und Diamine wurde gaschromatographisch, durch Vergleich mit chemisch reinen Substanzen bestimmt.

## Patentansprüche

1. Verfahren zur Herstellung primärer Amine aus Aldehyden in Gegenwart eines Verdünnungsmittels, Ammoniaks, wobei pro Mol Aldehydgruppe mindestens 15 Mol Ammoniak verwendet werden, eines Hydrierkatalysators, Wasserstoffs und Isolierung des gebildeten Amins aus dem Reaktionsgemisch, dadurch gekennzeichnet, daß ein Aldehyd mit einem Verdünnungsmittel, wobei im Falle eines Alkohols oder von Wasser als Verdünnungsmittel die Mischungstemperatur höchstens 5°C beträgt, gemischt wird, sodaß sich kein Hemiacetal oder Aldehydhydrat bildet und die Mischung unmittelbar danach, praktisch gleichzeitig mit Ammoniak, Wasserstoff und dem Hydrierkatalysator bei Temperaturen von 60 bis 180°C und Drücken von 20 bis 60 bar in Kontakt gebracht wird.

2. Verfahren nach Anspruch 1, dadurch gekennzeichnet, daß als Verdünnungsmittel ein Alkohol mit 1 bis 8 C-Atomen eingesetzt wird.

3. Verfahren nach Anspruche 2, dadurch gekennzeichnet, daß eine Mischungstemperatur des Aldehyds und des Alkohols oder des Aldehyds und des Wassers von -5 bis 0 °C angewandt wird.

4. Verfahren nach einem der Ansprüche 1 bis 3, dadurch gekennzeichnet, daß als Aldehyd ein Alkanal, Alkenal, Alkandial oder Alkendial mit 4 bis 22 C-Atomen oder ein unsubstituierter oder durch Hydroxy- oder Alkoxygruppen mit 1 bis 6 C-Atomen substituierter Benzaldehyd, ein Pyrrol- oder ein Pyridinaldehyd eingesetzt wird.

5. Verfahren nach einem der Ansprüche 1 bis 4, dadurch gekennzeichnet, daß eine Reaktionstemperatur von 80 bis 130 °C eingehalten wird.

6. Verfahren nach einem der Ansprüche 1 bis 5, dadurch gekennzeichnet, daß als Hydrierkatalysator ein Nickelkatalysator eingesetzt wird.

7. Verfahren nach einem der Ansprüche 1 bis 6, dadurch gekennzeichnet, daß die Reaktion unter einem Druck von 40 bis 60 bar durchgeführt wird.

8. Vorrichtung zur kontinuierlichen Herstellung primärer Amine aus Aldehyden, dadurch gekennzeichnet, daß ein Mischbehälter M, der gekühlt werden kann und der eine Eintragsvorrichtung für einen Aldehyd A und ein Verdünnungsmittel LM und eine Austragsvorrichtung für das Verdünnungsmittel oder die Mischung aus Aldehyd und Verdünnungsmittel enthält, mit einem Reaktionsgefäß RK, das drucksicher und beheizbar ist, eine Durchmischungsvorrichtung R und Zuleitungen für Ammoniak NH₃ und Wasserstoff H₂ enthält, mittels einer Leitung verbunden ist, wobei das Reaktionsgefäß eine Austragsvorrichtung für das ausreagierte Reaktionsgemisch RM enthält, das einen Austrag des Katalysators K verhindert, und wobei das Reaktionsgefäß RK gegebenenfalls mit einer Destillationsanlage D mittels einer Leitung verbunden ist, in der das im Reaktionsgefäß gebildete Amin vom Ammoniak, gegebenenfalls Wasserstoff und dem Verdünnungsmittel LM abgetrennt werden kann, wobei die Destillationsanlage D mittels einer Leitung, in der das abgetrennte Ammoniak und der Wasserstoff befördert werden können, mit dem Reaktionsgefäß RK und mittels einer Leitung in der das abgetrennte, gegebenenfalls gereinigte Verdünnungsmittel befördert werden kann, mit dem Mischungsbehälter M verbunden ist.

9. Verfahren nach einem der Ansprüche 1 bis 7, dadurch gekennzeichnet, daß eine Vorrichtung gemäß Anspruch 8 eingesetzt wird.

10. Verfahren nach Anspruch 9, dadurch gekennzeichnet, daß die Konzentration des Aldehyds im Reaktionsgefäß RK durch kurzzeitiges Stoppen der Aldehydzufuhr und Eintragung von reinem Verdünnungsmittel und gegebenenfalls Ammoniak kontinuierlich verändert wird.
